(19) Europäisches Patentamt
European Patent Office
Office européen des brevets

(11) EP 2 153 816 B1

(12) EUROPEAN PATENT SPECIFICATION

(45) Date of publication and mention of the grant of the patent:
**07.10.2015 Bulletin 2015/41**

(51) Int Cl.:
***A61K 8/44*** *(2006.01)*
***A61K 8/86*** *(2006.01)*
***A61Q 5/02*** *(2006.01)*
***A61Q 19/10*** *(2006.01)*
***A61K 8/46*** *(2006.01)*

(21) Application number: **09005272.1**

(22) Date of filing: **11.04.2009**

(54) **Shear thickening cleansing composition**

Scherverdickende Reinigungszusammensetzung

Composition de nettoyage d'épaississement par cisaillement

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **15.08.2008 EP 08007489**

(43) Date of publication of application:
**17.02.2010 Bulletin 2010/07**

(73) Proprietor: **Kao Germany GmbH**
**64297 Darmstadt (DE)**

(72) Inventors:
- **Hoffmann, Martin**
  **64673 Zwingenberg (DE)**
- **Leukel-Schäfer, Diana**
  **64297 Darmstadt (DE)**

(74) Representative: **Grit, Mustafa**
**KPSS - Kao Professional Salon Services GmbH**
**Pfungstädterstraße 92-100**
**64297 Darmstadt (DE)**

(56) References cited:
**WO-A-97/40125**
**WO-A-03/013459**
**WO-A-03/013467**
**WO-A-03/063818**
**WO-A-2004/024110**
**WO-A-2005/048971**
**WO-A-2006/065878**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention is related to use of an ethoxylated monoglyceride in an aqueous cleansing composition for keratin fibres, especially human hair, as a shear thickening agent.

Cleansing compositions have been known for many years. Many patent applications and scientific publications deal with such compositions aiming at cleansing and especially improved conditioning effects. One of the requirements for a precise application of a cosmetic composition is that it should have certain consistency. Consistency of such composition must make optimal application at the site of usage possible with optimal dosage. Therefore, usually thickening agents are used for adjusting consistency of cosmetic compositions.

There are plenty of thickening agents available especially for cleansing compositions. Although the prior art developed quite extensively, it will make the formulators job easier in case a cleansing composition is thickened itself when pumping from a dispenser or pouring from a bottle or a tube under certain level of shear stress. In other words, it is a great advantage for developers of cleansing compositions, when a cleansing composition having its own consistency, but not the consistency required for optimal dosage and application, is thickened when taken out from a tube or a bottle by squeezing the tube or bottle strongly or when pumped out from a dispenser with relatively high shear stress.

WO 03/013459 A2 and WO 97/40125 A1 disclose cleansing compositions based on anionic and aminoacid surfactants and further comprising ethoxylated monoglyceride. Both documents do not menation anything on shear thickening effects of ethcoylated monoglyceride.

Present inventors have surprisingly found that ethoxylated monoglyceride show excellent shear thickening property in a cleansing composition comprising at least one anionic surfactant, at least one amino acid surfactant.. This property is referred as rheopex behaviour in the rheology literature.

Accordingly, the first object of the present invention is the use of at least one ethoxylated monoglyceride of the following formula

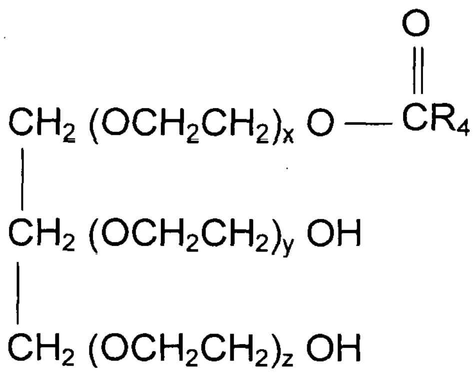

wherein $R_4$ is a saturated or unsaturated and branched or straight alkyl chain with a chain length of 7 to 21 C atoms and x+y+z has a value of 50 to 200, at a concentration of at least 1% by weight, in a cleansing composition based on at least one sulphate type of anionic surfactant, at a concentration of 2 to 25% by weight, and further comprising at least one amino acid surfactant of the following structure

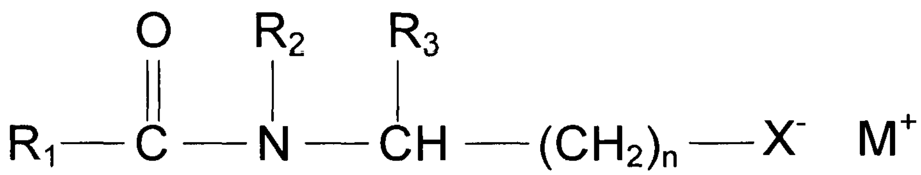

wherein $R_1$ is a saturated or unsaturated, straight or branched alkyl chain with 7 to 17 C atoms, $R_2$ is H or a methyl, $R_3$ is H, $COO^- M^+$ or $CH_2COO^- M^+$, n is 0 to 2, X is $COO^-$ or $SO_3^-$ and $M^+$ is independent from each other H, sodium or potassium, at a concentration of 0.1 to 15%, by weight, all values calculated to total composition, as a shear thickening agent.

Within the meaning of the present invention, with the term anionic surfactant, anionic surfactants are meant other than those of amino acid surfactants.

With the term amino acid surfactants especially those surfactants are meant derived from taurate, glucamate, alanin or alaninate, sarcosinate and aspartate.

With the term shear thickening, it is meant that composition has a lower consistency at zero or low shear stress

than in presence of shear stress or higher shear stress values. In other words, composition has a lower consistency in an unmixed state than when it is mixed with for example spatula in a beaker.

Cleansing composition in the use of the present invention comprises at least one ethoxylated monoglyceride according to the above general formula. In the preferred embodiment of the present invention $R_1$ is a saturated or unsaturated and branched or straight alkyl chain with a chain length of 11 to 17 C atoms, more preferably 13 to 17 C atoms and most preferably 15 to 17 C atoms and x+y+z has preferably a value of 60 to 200, more preferably 70 to 200 and most preferably 80 to 200.

Ethoxylated monogylcerides are known for their thickening ability in the area of hair cleansing compositions. For example WO 03/ 063818 A1 discloses ethoxylated glycerides as thickening agents in combination with ethoxylated fatty alcohol and ethoxylated partial gylcerides. WO 2004/024110 A1 and WO 03/013467 A1 disclose cleansing compositions comprising an ethoxylated monogylceride. However, all three patent publications do not mention shear thickening property of ethoxylated monoglycerides according to the above disclosed general formula in cleansing compositions.

Non-limiting suitable examples of ethoxylated monoglycerides are PEG-78 glyceryl cocoate, PEG-80 glyceryl cocoate, PEG-50 glyceryl isostearate, PEG-60 glyceryl isostearate, PEG-90 glyceryl isostearate, PEG-60 glyceryl stearate, PEG-120 glyceryl stearate, PEG-200 glyceryl stearate, PEG-80 glyceryl tallowate, PEG-82 glyceryl tallowate, PEG-130 glyceryl tallowate, PEG-200 glyceryl tallowate, and mixtures thereof. Preferred are PEG-78 glyceryl cocoate, PEG-80 glyceryl cocoate, PEG-60 glyceryl isostearate, PEG-90 glyceryl isostearate, PEG-60 glyceryl stearate, PEG-120 glyceryl stearate, PEG-200 glyceryl stearate, PEG-80 glyceryl tallowate, PEG-82 glyceryl tallowate, PEG-130 glyceryl tallowate and PEG-200 glyceryl tallowate. More preferred are PEG-78 glyceryl cocoate, PEG-80 glyceryl cocoate, PEG-90 glyceryl isostearate, PEG-120 glyceryl stearate, PEG-200 glyceryl stearate, PEG-80 glyceryl tallowate, PEG-82 glyceryl tallowate, PEG-130 glyceryl tallowate, PEG-200 glyceryl tallowate, and mixtures thereof. Most preferred are PEG-80 glyceryl cocoate, PEG-90 glyceryl isostearate, PEG-120 glyceryl stearate, PEG-200 glyceryl stearate, PEG-80 glyceryl tallowate, PEG-82 glyceryl tallowate, PEG-130 glyceryl tallowate, PEG-200 glyceryl tallowate, and mixtures thereof. The especially preferred ethoxylated monoglyceride is PEG-90 glyceryl isostearate which is available from Zschimmer & Schwarz under the trade name Oxetal VD 92.

Concentration of ethoxylated monoglyceride in the use of the present invention is preferably in the range of 1 to 15%, more preferably 1.5 to 12.5%, most preferably 1.5 to 7.5% and especially 2 to 5% by weight, calculated to total composition.

Cleansing composition in the use of the present invention comprises at least one amino acid surfactant according to the general formula given above at a concentration of 0.1 to 15%, by weight, calculated to total composition. Preferably, the concentration of amino acid surfactant is from 0.25 to 10% by weight, more preferably 0.5 to 7.5% by weight and most preferably 1 to 5% by weight, calculated to total composition. In the preferred embodiment of the present invention $R_1$ in the general formula of amino acid surfactants disclosed above is a saturated or unsaturated, straight or branched alkyl chain with 9 to 17 C atoms, and more preferably 9 to 13 C atoms, $R_2$ is H or a methyl, $R_3$ is H, $COO^- M^+$, $CH_2COO^-$ M or COOH, n is 0 to 2, X is $COO^-$ or $SO_3^-$ and M is independent from each other H, sodium or potassium. It should be noted that alkyl chain includes also mixture of various alkyl groups as present especially in plant triglyceride derived alkyl chains such as cocoyl chain.

Suitably amino acid surfactant types are taurate, glutamate, alanin or alaninate, sarcosinate, aspartate surfactants, and mixtures thereof. Preferred are taurate, glutamate and sarcosinate surfactants and mixtures thereof. More preferred are taurates and glutamates and most preferred is glutamate type surfactants.

Suitable taurate surfactants are according to the general formula

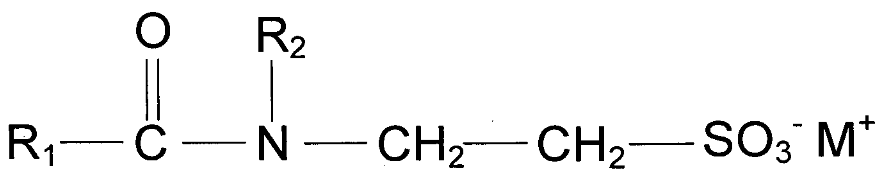

wherein $R_1$ is preferably a saturated or unsaturated, straight or branched alkyl chain with 7 to 17 C atoms, and more preferably 9 to 13 C atoms, $R_2$ is H or methyl, and M is H, sodium or potassium. Suitable examples are potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, and sodium methyl stearoyl taurate and mixtures thereof. Preferred are potassium cocoyl taurate, potassium methyl cocoyl taurate, sodium caproyl methyl taurate, sodium cocoyl taurate, sodium lauroyl taurate, sodium methyl cocoyl taurate and sodium methyl lauroyl taurate and mixtures thereof. More preferred are potassium cocoyl taurate, potassium methyl cocoyl taurate, sodium cocoyl taurate, sodium lauroyl taurate, sodium

methyl cocoyl taurate and sodium methyl lauroyl taurate and mixtures thereof.

Suitable glutamate surfactants are according to the general formula

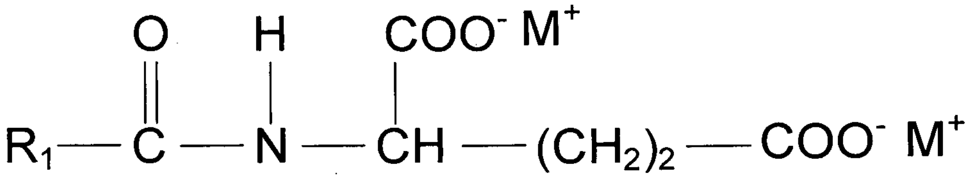

wherein $R_1$ is preferably a saturated or unsaturated, straight or branched alkyl chain with 7 to 17 C atoms, and more preferably 9 to 13 C atoms, and M is independent from each other H, sodium or potassium. Suitable examples are dipotassium capryloyl glutamate, dipotassium undecylenoyl glutamate, disodium capryloyl glutamate, disodium cocoyl glutamate, disodium lauroyl glutamate, disodium stearoyl glutamate, disodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, and sodium undecylenoyl glutamate and mixtures thereof. Preferred are disodium capryloyl glutamate, disodium cocoyl glutamate, disodium lauroyl glutamate, potassium capryloyl glutamate, potassium cocoyl glutamate, potassium lauroyl glutamate, potassium myristoyl glutamate, sodium capryloyl glutamate, sodium cocoyl glutamate, sodium lauroyl glutamate, and sodium myristoyl glutamate and mixtures thereof. More preferred are disodium capryloyl glutamate, disodium cocoyl glutamate, disodium lauroyl glutamate, potassium capryloyl glutamate, potassium cocoyl glutamate, potassium lauroyl glutamate, sodium capryloyl glutamate, sodium cocoyl glutamate, and sodium lauroyl glutamate and mixtures thereof.

Suitable alanine or alaninate surfactants are according to the general formula

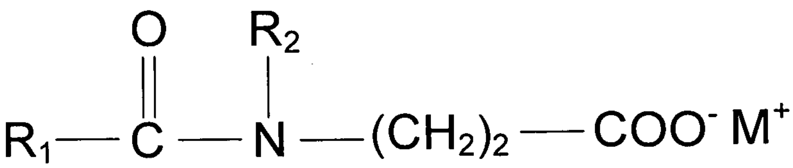

wherein $R_1$ is preferably a saturated or unsaturated, straight or branched alkyl chain with 7 to 17 C atoms, and more preferably 9 to 13 C atoms, $R_2$ is H or methyl and M is H, sodium or potassium. Suitable examples are cocoyl methyl β-alanine, lauroyl ß-alanine, lauroyl methyl ß-alanine, myristoyl ß-alanin, potassium lauroyl methyl ß-alanine, sodium cocoyl alaninate, sodium cocoyl methyl β-alanine and sodium myristoyl methyl ß- alanine and mixtures thereof.

Suitable glycine surfactants are according to the general formula

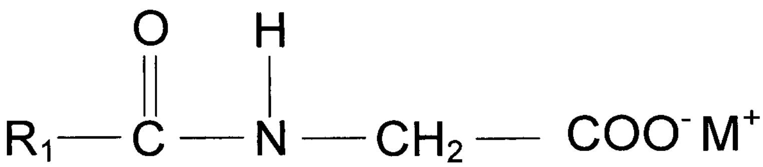

wherein $R_1$ is preferably a saturated or unsaturated, straight or branched alkyl chain with 7 to 17 C atoms, and more preferably 9 to 13 C atoms, and M is H, sodium or potassium. Suitable examples are palmitoyl glycine, sodium lauroyl glycine, sodium cocoyl glycine, sodium myristoyl glycine, potassium lauroyl glycine, and potassium cocoyl glycine and mixtures thereof.

Suitable sarcosinate surfactants are according to the general formula

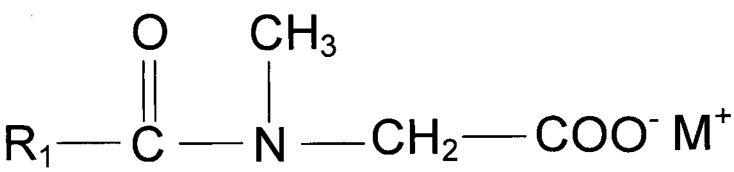

wherein $R_1$ is preferably a saturated or unsaturated, straight or branched alkyl chain with 7 to 17 C atoms, and more preferably 9 to 13 C atoms, and M is H, sodium or potassium. Suitable examples are potassium lauroyl sarcosinate, potassium cocoyl sarcosinate, sodium cocoyl sarcosinate, sodium lauroyl sarcosinate, sodium myristoyl sarcosinate, and sodium palmitoyl sarcosinate and mixtures thereof. Preferred are potassium lauroyl sarcosinate, potassium cocoyl sarcosinate, sodium cocoyl sarcosinate, and sodium lauroyl sarcosinate and mixtures thereof. More preferred are sodium cocoyl sarcosinate, and sodium lauroyl sarcosinate and mixtures thereof.

Suitable aspartate surfactants are according to the general formula

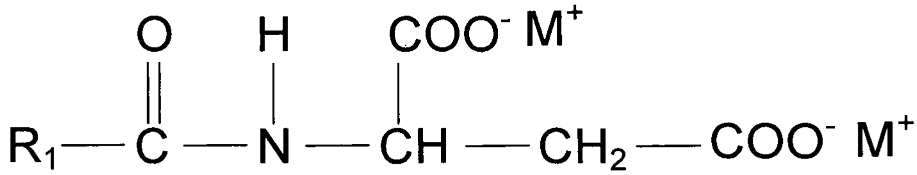

wherein $R_1$ is preferably a saturated or unsaturated, straight or branched alkyl chain with 7 to 17 C atoms, and more preferably 9 to 13 C atoms, and M is independent from each other H, sodium or potassium. Suitable examples are sodium lauroyl aspartate, sodium myristoyl aspartate, sodium cocoyl aspartate, sodium caproyl aspartate, disodium lauroyl aspartate, disodium myristoyl aspartate, disodium cocoyl aspartate, disodium caproyl aspartate, potassium lauroyl aspartate, potassium myristoyl aspartate, potassium cocoyl aspartate, potassium caproyl aspartate, dipotassium lauroyl aspartate, dipotassium myristoyl aspartate, dipotassium cocoyl aspartate, and dipotassium caproyl aspartate and mixtures thereof. Preferred are sodium lauroyl aspartate, sodium myristoyl aspartate, sodium cocoyl aspartate, and sodium caproyl aspartate and mixtures thereof.

It should be noted that compositions in the use of the present invention can also comprise mixture of several type of amino acid surfactants such as mixture of glutamate and taurate surfactants, or mixture of taurate, glutamate and sarcosinate surfactants etc.

Cleansing compositions in the use of the present invention comprise at least one anionic sulphate type of surfactant at a concentration range of 2 to 25%, preferably 2.5 to 20% and more preferably 5 to 15%, and most preferably 7.5 to 15% by weight, calculated to the total composition.

The most preferred anionic surfactants within the meaning of the present invention are those of alkyl ether sulphates such as lauryl ether sulphate sodium salt.

In a preferred embodiment of the present invention, cleansing composition in the ouse of the present invention comprises at least one anionic surfactant as mentioned above and at least one nonionic surfactant. Nonionic surfactants are suitable at a concentration of 1 % to 15 %, in particular from 1 % to 10 % by weight, calculated to the total composition.

Nonionic surfactants especially suited in the cleansing compositions according to the invention are alkyl polyglucosides of the general formula

$$R_6\text{-}O\text{-}(R_4O)_n\text{-}Z_x,$$

wherein $R_6$ is an alkyl group with 8 to 18 carbon atoms, $R_4$ is an ethylene or propylene group, Z is a saccharide group with 5 to 6 carbon atoms, n is a number from 0 to 10 and x is a number between 1 and 5.

These alkyl polyglucosides are known in particular as excellent skin-compatible, foam improving agents in liquid detergents and body cleansing compositions. Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactants are, suitable for the cleansing compositions of the present invention, long-chain fatty acid dialkanolamides, such as coco fatty acid diethanolamide and myristic fatty acid diethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics$^R$", as well as fatty alcohol ethoxylates.

Further suitable nonionic surfactants are amineoxides which may be present in an amount from 0.25 % to 5 % by weight, calculated to the total composition. Such amineoxides are state of the art, for example $C_{12}$-$C_{18}$- alkyl dimethyl amineoxides such as lauryl dimethyl amineoxide, $C_{12}$-$C_{18}$- alkyl amidopropyl or -ethyl amineoxides, $C_{12}$-$C_{18}$ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx®", "Aromox®", or "Genaminox®".

Further nonionic surfactants useful in the compositions according to invention are $C_{10}$-$C_{22}$-fatty alcohol ethox-

ylates at a concentration of 0.5 to 10%, preferably 0.5 to 5% by weight, calculated to total composition. Especially suited are $C_{10}$-$C_{22}$-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

The most preferred non-ionic surfactants are alkyl polyglucosides such as decyl, cocoyl polyglucoside and ethoxylated fatty alcohols such as laureth-16.

In a further preferred embodiment of the present invention, cleansing composition in the use of the present invention comprises at least one anionic, at least one nonionic surfactant and at least one amphoteric or zwitterionic surfactant.

Amphoteric or zwitterionic surfactants, are present at a concentration of 0.5 % to about 15 %, preferably 1 % to about 10 %, by weight, calculated to the total composition. It has especially been found out that addition of zwitterionic or amphoteric surfactants enhances foam feeling in terms of creaminess, foam volume and as well as skin compatibility are also improved. For achieving milder formulations anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, suitable betaine surfactants are of general structure

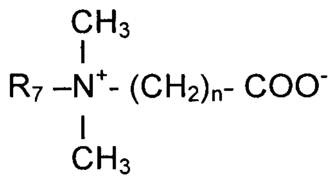

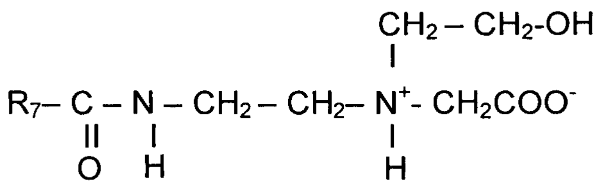

wherein $R_7$ is a $C_8$-$C_{18}$-alkyl group and n is 1 to 3;
sulfobetaines of the structure

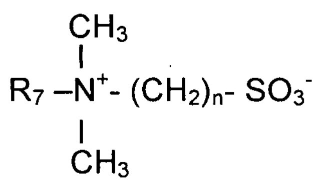

wherein $R_7$ and n are same as above;
and amidoalkyl betaines of the structure

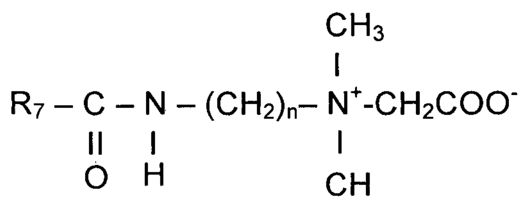

wherein $R_7$ and n are same as above.

The most preferred amphoteric surfactants are alkyl betaines such as lauryl betaine and alkyl amido betaines such as cocamidopropyl betaine.

In a further preferred form of the present invention, cleansing composition comprises at least one anionic surfactant of alkyl ether sulphate type, at least one amphoteric surfactant especially alkyl amido alkyl betaine type and at least one non-ionic surfactant especially an alkyl polyglucoside.

In another preferred form of the present invention cleansing compositions comprise additionally at least one (poly)propylene glycol according to the following formula

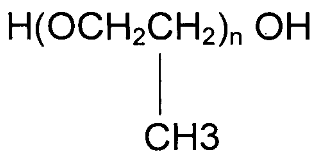

wherein n has a value between 1 and 70. It has been observed additionally that cleansing composition comprising at least one (poly)propylene glycol show excellent volume up effect especially for fine hair.

Non-limiting suitable examples are PPG-3, PPG-7, PPG-9, PPG-12, PPG-13, PPG-15, PPG-16, PPG-17, PPG-20, PPG-26, PPG-30, PPG-33, PPG-34, PPG-51 and PPG-69. Preferred are PPG-3, PPG-7, PPG-9, PPG-12, PPG-13, PPG-15, PPG-16, PPG-17, PPG-20, PPG-26, PPG-30, PPG-33 and PPG-34 and mixtures thereof. More preferred polypropylene glycols are PPG-7, PPG-9, PPG-12, PPG-13, PPG-15, PPG-16, PPG-17, PPG-20, PPG-26 and PPG-30 and mixtures thereof. Most preferred ones are PPG-7, PPG-9, PPG-12, PPG-13, PPG-15, PPG-16, PPG-17 and PPG-20 and mixtures thereof. Especially preferred one is PPG-9 with which most of the experiments were carried out.

Concentration of at least one (poly)propylene glycol of the above formula in compositions of the present invention is in the range of 0.1 to 10%, more preferably 0.25 to 7.5%, and most preferably 0.5 to 5% by weight, calculated to total composition.

In a further preferred embodiment, cleansing composition in the use of the present invention comprises hair-conditioning agents. Conditioning agents can be selected from oily substances, non-ionic substances, cationic amphiphilic ingredients, cationic polymers or their mixtures.

Oily substances are selected from such as silicone oils, either volatile or non-volatile, natural and synthetic oils. Among silicone oils those can be added to the compositions include dimethicone, dimethiconol, polydimethylsiloxane, DC fluid ranges from Dow Corning, arylated silicones such as phenyl trimethicone or any other silicone with up to 5 aryl, preferably phenyl, group in its molecule, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents can be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

$$R_8CO\ (O\ CH_2\ CH_2)_nOH$$

or

$$R_8CO\ (OCH_2\ CH_2)_nO\ OC\ R_9$$

where $R_8$ and $R_9$ are independent from each other saturated, unsaturated or branched or non-branched alkyl chain with 7 to 21 C atoms and n is typically 2-100.

In one of the preferred form of the present invention, cleansing compositions comprise at least one cationic polymer as conditioning agent. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium

59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87. As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

Although less preferred, cleansing compositions of the present invention may comprise additionally one or more cationic surfactant(s) as conditioner presented with the general formula

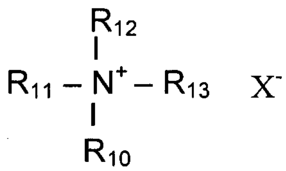

where $R_{10}$ is a saturated or unsaturated, branched or non-branched alkyl chain with 8-22 C atoms or

$R_{14}CO\ NH\ (CH_2)_n$

where $R_{14}$ is saturated or unsaturated, branched or non-branched alkyl chain with 7-21 C atoms and n has value of 1 - 4, or

$R_{15}CO\ O\ (CH_2)_n$

where $R_{15}$ is saturated or unsaturated, branched or non-branched alkyl chain with 7-21 C atoms and n has value of 1 - 4, and

$R_{11}$ is hydrogen or unsaturated or saturated, branched or non-branched alkyl chain with 1 - 22 C atoms or

$R_{14}\ CO\ NH\ (CH_2)_n$

or

$R_{15}CO\ O\ (CH_2)_n$

where $R_{14}$, $R_{15}$ and n are same as above.

$R_{12}$ and $R_{13}$ are hydrogen or lower alkyl chain with 1 to 4 carbon atoms, and X is anion such as chloride, bromide, methosulfate.

Typical examples of those ingredients are cetyl trimethly ammonium chloride, stear trimonium chloride, behentrimonium chloride, stearamidopropyl trimonuim chloride, dioleoylethyl dimethyl ammonium methosulfate, dioleoylethyl hydroxyethylmonium methosulfate.

The compositions according to the invention may also comprise further conditioning substances such as protein hydrolyzates and polypeptides, e.g., keratin hydrolyzates, collagen hydrolyzates of the type "Nutrilan$^R$" or elastin hydrolyzates, as well as also in particular plant protein hydrolyzates, optionally, cationized protein hydrolyzates, e.g., "Gluadin$^R$".

Typical concentration range for any of those conditioners of cationic polymers, silicon oil and derivatives and cationic surfactants can be 0.01 - 5% by weight, preferably 0.01 - 3.5% by weight, more preferably 0.05 - 2.5% and most preferably 0.1 - 1.5% by weight calculated to the total composition. Most preferred conditioning agents are cationic polymers.

Further conditioning additives are hair conditioning and/or styling polymers. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning shampoo composition of the present invention. They are incorporated alone or in admixture with at least one additional cationic, nonionic or anionic polymer, particularly copolymers of N-octyl acrylamide, (meth)acrylic acid and tert-butyl aminoethyl methacrylate of the type "Amphomer®"; copolymers from methacryl oylethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl aminoalkyl (meth)acrylamides containing basic groups, in particular amino groups; copolymers from N-octyl acrylamide, methyl methacrylate, hydroxypropyl methacrylate, N-tert.-butyl aminoethyl - methacrylate and acrylic acid, as well as the copolymers known from US-A 3,927,199, are applicable.

Cleansing composition of the present invention can be transparent as well as pearly. Transparency of the composition is judged by naked eye in a transparent shampoo bottle with a thickness not more than 5 cm. In the case a transparent appearance is wished, the following ingredients are not essential. Pearl-shiny appearance is achieved with those dispersed in cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kinds of mixtures are available commercially.

Solubilizers may be added to the compositions especially when oily substances are chosen as conditioning agents and fragrance oils with highly lipophilic properties. Typical solubilizers may be hydrogenated castor oil known with the trade mark Cremophor CO series from BASF. It should be noted that as well the surfactant mixture can be a good solubilizer for fragrance oils. Typical concentration of the solubilizers can be in the range of 0.01 - 2% by weight, preferably 0.1 - 1 % by weight, calculated to total composition.

The cleansing composition may contain active ingredients selected from UV fitters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are preferably selected from polycarboxy acids. The preferred one is ethylene diamine tetraacetic acid, EDTA. Typical useful concentration range for sequestering agents is of 0.01 - 2.5% by weight calculated to the total composition.

The UV filters are that oil and water soluble ones for the purpose of protecting hair colour. In other words, anionic and nonionic, oily, UV filters are suitably used in the compositions of the present invention. Suitable UV-absorbing substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy-benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzylidenecampher, 3-(4'sulfo)-benzyl-idenebornane-2-one and the salts thereof, 3-(4'-methyl benzylidene)-DL-campher, and/or polysilicone-15. The amount of the UV-absorber ranges typically from about 0.01 % to 2.5%, more preferably from 0.05 % to 1 % by weight, calculated to the total composition.

Natural plant extracts are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits,

blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, various "Extrapone®" products, and "Herbasol$^R$". Extracts and the preparation thereof are also described in "Hagers Handbuch der pharmazeutischen Praxis", 4$^{th}$ Ed..

Compositions of the present invention may comprise further at least one compound according to the formula

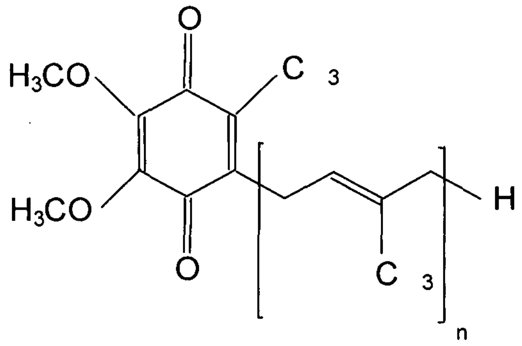

where n is a number from 1 to 10.

The compounds of the above formula are known as Ubiquinone, and also are known as Coenzyme. It should be noted that the compositions of the present invention can certainly comprise more than one ubichinone. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Cleansing compositions of the present invention can also comprise synthetic mica as a further shine enhancer.

Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail, the content of the document is included herewith by reference. It also discloses a cleansing composition comprising monoethanolamide surfactant in addition to other surfactants.

Suitable metal oxide or oxides for coating synthetic mica are titanium dioxide, chromium oxide, ferric oxide or mixtures thereof. In the present invention the preferred is synthetic mice coated with titanium dioxide. Such materials are commercially available from Sun Chemical Corporation and known with their INCI names Synthetic Fluorphologopite.

The particle size distribution of synthetic mica coated with a metal oxide or oxides is in the range of 1 to 750 $\mu$m, preferably 1 to 250 $\mu$m, more preferably 1 to 100 $\mu$m and most preferably 20 to 95 $\mu$m. The particle sizes referred are relating to the volume particle size distribution meaning that particles found in the coated synthetic mica having volume particle size in the given ranges.

Concentration of synthetic mica coated with at least metal oxide or oxides is from 0.001 to 10%, preferably 0.05 to 7.5%, more preferably 0.1 to 5% and most preferably 0.20 to 2.5% by weight calculated to total composition.

Further in a preferred embodiment of the present invention, compositions comprise at least one direct dye. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuff categories is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87, and mixtures thereof

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow

1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and mixtures thereof.

Among those, the preferred anionic dyestuffs are Acid Red 52, Acid Violet 2, Acid Red 33, Acid Orange 4, Acid Red 27 and Acid Yellow 10 and their salts, and mixtures thereof. The most preferred anionic dyes are Acid Red 52, Acid Violet 2, Acid Red 33, Acid Orange 4 and Acid Yellow 10, and their salts, and mixtures thereof.

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1. HC Brown No.2, HC Green No.1, HC Orange No.1, HC Orange No.2, HC Orange No.3, HC Orange No 5, HC Red BN, HC Red No.1, HC Red No.3, HC Red No.7, HC Red No.8, HC Red No.9, HC Red No.10. HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10. HC Yellow No.11, HC Yellow No. 12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and mixtures thereof.

Concentration of one or more direct dyes in total is in the range of 0.001 to 5% by weight, preferably 0.01 to 4% more preferably 0.05 to 3% and most preferably 0.1 to 2.5% by weight calculated to total composition. The most preferred among the direct dyes is cationic direct dyes.

It is self-understood that the shampoos according to the invention may comprise other substances customarily used in such compositions such as preservatives, fragrances.

The pH of the compositions according to the present invention is suitably between 2 and 8.0, preferably in the range of 2.5 to 7.0, more preferably 3 to 6.5 and most preferably 4 to 5.5 measured at ambient temperature with a suitable pH meter.

pH of the compositions is adjusted with acidic and alkaline compounds. Acidic compounds can be inorganic and organic acid or their mixtures. Nonlimiting suitable examples are citric acid, lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Alkaline compounds such as sodium hydroxide can be used to adjust the pH of the compositions.

Cleansing compositions in the use of the present invention preferably has a viscosity value at zero or very low shear stress, due to viscosity measuring conditions, and at 20°C measured with Brookfield viscosimetre in the range of 1,000 to 60,000, preferably 2,000 to 50,000 and more preferably 5,000 to 40,000 mPa.s.

The following examples are to illustrate the invention, but not to limit. The products according to the invention are prepared by mixing the individual components in water, whereby it is also possible to use pre-mixtures of various ingredients.

**Example 1**

| | % by weight |
|---|---|
| Sodium lauryl ether sulphate | 12.6 |
| Sodium lauroyl glutamate | 1.5 |
| PEG-90 glyceryl isostearate | 3.0 |
| Citric acid/sodium hydroxide | q.s. to pH 5.5 |
| Preservative, fragrance | q.s |
| Water | to 100 |

For the comparative purposes the above composition was also prepared without PEG-90 glyceryl isostearate. In the comparative example 2% by weight sodium chloride is used as thickening agent. Viscosity of the both compositions measured at 20°C with a Brookfield viscosimetre with Spindle 93 at 5 rpm were approximately 35,000 mPa.s (35 Pa.s).

Rheopex behaviour of the cleansing compositions is determined according to the method disclosed in "Das Rheologie Handbuch, 2. Auflage, Menzer, T.G., Vincentz Network GmbH, Hannover, 2006, pages 246 - 250" at 20°C.

Briefly, the above compositions were filled into a cylinder shaped "syringe" like vessel with a diameter of 0.025

m (d) and with an opening diameter of 0.006 m ($d_3$). The composition filled into the vessel had a volume of 2.45 x $10^{-5}$ $m^3$ (V) and a height of 0.05 m ($L_1$). The height of opening was 0.02 m ($L_3$). Under a weight of 0.545 kg (m), the flow time for the complete emptying of the cleansing composition from the vessel was determined in seconds ($t_i$ = inventive composition and $t_c$ = comparative composition). The emptying times determined were $t_i$ = 77 s and $t_c$ = 12 s). With the help of the equations below the viscosity values were calculated in Pa.s.

$$F = m * g$$

wherein F is the force in N and m is the weight in kg and g is the gravity constant (9.8 m $s^{-2}$).

From the above equation a force of 5.35 N is calculated.

The pressure (p) at the opening was calculated with the following equation:

$$p = \frac{F}{A} = \frac{F}{r^2 \pi} = pressure$$

wherein p is pressure, A is the cross sectional area of the vessel in $m^2$ wherein the compositions were filled and r is radius in m.

The shear stress ($\tau$) at the opening was calculated with the following equation:

$$\tau = \frac{p * R_3}{2 * L_3} = \frac{F * R_3}{2 * L_3 * \left(\frac{d}{2}\right)^2 * \pi} = \frac{F * d_3}{L_3 * d^2 * \pi}$$

wherein $R_3$ is radius at the opening in m.

Form the above equation under the current measurement conditions, the value of $\tau$ was obtained to be 817.3 $Nm^{-2}$

The shear rate ($\gamma$) at the opening was calculated with the following equation:

$$\dot{\gamma} = \frac{4 * \dot{V}}{\pi * R_3^3} = \frac{4 * V}{\pi * R_3^3 * t}$$

Form the above equation, $\gamma$ value for the inventive composition ($\gamma$) with $t_i$ value of 77 s at the place of t was calculated to be 96.3 $s^{-1}$ and for the comparative composition ($\gamma_c$) with $t_c$ value of 12 s at the place of t was calculated to be 15.0 $s^{-1}$.

Viscosity values ($\eta$) during the shear stress are calculated using the following equation:

$$\eta = \frac{\tau}{\dot{\gamma}} = \left[\frac{Ns}{m^2} = Pas\right]$$

With the above mentioned $\tau$ and $\gamma_i$ and $\gamma_c$ values calculated for inventive composition and for comparative composition, respectively, the following viscosity values for inventive composition ($\eta_i$) and comparative composition ($\eta_c$) were calculated:

$$\eta_I = 54.4 \text{ Pa.s}$$

$$\eta_c = 8.5 \text{ Pa.s}$$

From the above it is clear that the inventive composition is a shear thickening composition, whereas the comparative composition is a shear thinning composition when for both compositions viscosity values measured with Brookfield viscosimetre (35 Pa.s) are taken into account with practically zero or negligibly very low shear stress wneh compared to the values used above.

Similar results were observed with the examples below.

**Example 2**

| | % by weight |
|---|---|
| Sodium lauryl ether sulphate | 9.0 |
| Cocyl glucoside | 4.0 |
| Sodium lauroyl glutamate | 2.0 |
| Polyquaternium-7 | 1.0 |
| PEG-90 glyceryl isostearate | 2.0 |
| Citric acid/sodium hydroxide | q.s. to pH 5.5 |
| Preservative, fragrance | q.s |
| Water | to 100 |

The above composition has excellent creamy rich foam and conditions hair excellently in terms of combability and soft hair feeling.

**Example 3**

| | % by weight |
|---|---|
| Sodium lauryl ether sulfate | 9.0 |
| Cocyl glucoside | 3.0 |
| Cocoyl betaine | 2.0 |
| Sodium lauroyl glutamate | 2.0 |
| Polyquaternium-6 | 0.5 |
| Dimethicone | 0.5 |
| Ubiquinone | 0.1 |
| PEG-90 glyceryl isostearate | 3.0 |
| PPG-9 | 2.0 |
| Citric acid/sodium hydroxide | q.s. to pH 5.0 |
| Preservative, fragrance | q.s |
| Water | to 100 |

The above composition improves hair volume, gives hair more elasticity in addition to the excellent creamy foam and conditioning effect in terms of combability, shine and soft hair feeling.

The above composition was tested with 10 female users. For comparative purposes, a composition not comprising PEG-90 gylceryl isosterate was prepared which was thickened with 2% sodium chloride. Both compositions had a viscosity of approximately 20,000 mPa.s measured with Brookfield viscosimetre at 20°C. Both compositions were filled in a tube with an opening diameter of 2 mm. The volunteers were asked to get out enough amount of shampoo from the tube for one hair wash as they usually do and pay attention to the consistency. All 10 volunteers preferred the consistency of the inventive composition because of its thicker consistency and gel like behaviour when freshly squeezed from the tube. Furthermore, it was found to be interesting that the shampoo composition became less thick and spreads on the hand surface. Comparative composition was found to be spreading on hand too quickly. At the same time, all volunteers said that the composition is slightly more difficult to get out from the tube compared to the comparative composition. This shows clearly that under the same condition, inventive composition thickens when taken out from a tube with a small opening diameter.

**Example 4**

| | % by weight |
|---|---|
| Sodium lauryl ether sulphate | 8.0 |
| Cocoyl polyglucoside | 1.5 |
| Cocamidopropyl betaine | 4.0 |
| Sodium cocoyl glutamate | 2.0 |
| Polyquaternium-7 | 0.8 |
| Dimethicone | 0.5 |
| PEG-90 glyceryl isostearate | 5.0 |
| PPG-9 | 1.2 |
| Basic red 51 | 0.1 |
| Citric acid/sodium hydroxide | q.s. to pH 5.5 |
| Preservative, fragrance | q.s |
| Water | to 100 |

The above composition gives hair a red shine, and additionally delivers excellent conditioning effect in terms of more elasticity, combability, shine and soft hair feeling in addition to the excellent creamy foam.

**Example 5**

| | % by weight |
|---|---|
| Sodium lauryl ether sulfate | 9.0 |
| Laureth - 16 | 3.0 |
| Cocoyl betaine | 2.0 |
| Sodium cocoyl glutamate | 2.0 |
| Guarhydroxypropyltrimonium chloride | 1.0 |
| PEG-90 glyceryl isostearate | 2.0 |
| PPG-9 | 1.0 |
| Trimethyl pentaphenyl trisiloxane | 0.3 |
| Basic yellow 87 | 0.08 |
| Basic red 76 | 0.001 |
| Citric acid/sodium hydroxide | q.s. to pH 5.0 |
| Preservative, fragrance | q.s |
| Water | to 100 |

Excellent conditioning effects were observed in terms of volume, combability, elasticity and managabiliy and additionally an excellent golden blonde shine was observed on light blond hair.

**Example 6**

| | % by weight |
|---|---|
| Sodium lauryl ether sulfate | 9.0 |
| Cocyl glucoside | 3.0 |
| Lauryl betaine | 2.0 |
| Sodium cocoyl glutamate | 2.0 |
| Guarhydroxypropyltrimonium chloride | 1.0 |

(continued)

| | % by weight |
|---|---|
| PEG-90 glyceryl isostearate | 2.0 |
| PPG-9 | 1.0 |
| Trimethyl pentaphenyl trisiloxane | 0.3 |
| Basic red 51 | 0.1 |
| Basic orange 31 | 0.05 |
| Citric acid/sodium hydroxide | q.s. to pH 5.0 |
| Preservative, fragrance | q.s |
| Water | to 100 |

Excellent red shine were observed on medium blond hair.

**Example 7**

| | % by weight |
|---|---|
| Sodium lauryl ether sulfate | 9.0 |
| Laureth - 16 | 3.0 |
| Cocoyl betaine | 2.0 |
| Sodium cocoyl glutamate | 2.0 |
| Polyquaternium-10 | 1.0 |
| PEG-90 glyceryl isostearate | 3.5 |
| PPG-9 | 0.7 |
| Carbopol Aqua CC | 5.0 |
| Synthetic fluorphologopite* | 0.5 |
| Citric acid/sodium hydroxide | q.s. to pH 4.7 |
| Preservative, fragrance | q.s |
| Water | to 100 |

*: Synthetic fluorphologopite used is commercially available from Sun Chemical Corporation under the trade name SunShine Glitter White with a particle size distribution in the range of 20 to 95 μm.

The above composition delivered excellent volume and shine to dark blonde fine hair.

**Example 8**

| | % by weight |
|---|---|
| Sodium lauryl ether sulfate | 10.0 |
| Cocoyl betaine | 2.0 |
| Decyl glucoside | 1.5 |
| Sodium lauroyl glutamate | 4.0 |
| Quaternium 80 | 0.5 |
| Polyquaternium-7 | 0.2 |
| PEG-90 glyceryl isostearate | 3.5 |
| PPG-9 | 1.7 |
| Citric acid/sodium hydroxide | q.s. to pH 5.0 |
| Preservative, fragrance | q.s |
| Water | to 100 |

Above shampoo was found to be excellent volume giving shampoo to fine hair in a monadic test.

**Example 9**

| | % by weight |
|---|---|
| Sodium lauryl ether sulphate | 8.0 |
| Cocoyl polyglucoside | 2.0 |
| Cocamidopropyl betaine | 4.0 |
| Sodium cocoyl glutamate | 2.0 |
| Trimethyl pentaphenyl trisiloxane | 0.3 |
| Polyquaternium-7 | 1.0 |
| PEG-120 glyceryl stearate | 3.0 |
| PPG-15 | 1.7 |
| Citric acid/sodium hydroxide | q.s. to pH 5.2 |
| Preservative, fragrance | q.s |
| Water | to 100 |

Above shampoo was found to be excellent in volume enhancing effect. Additionally it improves combability and showed excellent shine enhancing effect.

**Example 10**

| | % by weight |
|---|---|
| Sodium lauryl ether sulphate | 8.0 |
| Cocoyl glucoside | 5.0 |
| Cocamidopropyl betaine | 4.0 |
| Sodium cocoyl glutamate | 2.0 |
| Polyquaternium-7 | 1.0 |
| PEG-90 glyceryl isostearate | 1.5 |
| PEG-30 glyceryl isostearate | 1.5 |
| PPG-15 | 0.3 |
| PPG-9 | 0.8 |
| Citric acid/sodium hydroxide | q.s. to pH 5.2 |
| Preservative, fragrance | q.s |
| Water | to 100 |

The above composition increases hair volume, improves combability and shine.

**Example 11**

| | % by weight |
|---|---|
| Sodium lauryl ether sulphate | 5.0 |
| Sodium lauryl ether carboxylate | 3.0 |
| Laureth - 16 | 3.0 |
| Cocoyl betaine | 2.0 |
| Sodium lauroyl glutamate | 2.0 |
| Polyquaternium-6 | 0.5 |

(continued)

| | % by weight |
|---|---|
| Dimethicone | 0.5 |
| Ubiquinone | 0.1 |
| PEG-60 glyceryl isostearate | 4.0 |
| PPG-9 | 2.0 |
| Citric acid/sodium hydroxide | q.s. to pH 5.0 |
| Preservative, fragrance | q.s |
| Water | to 100 |

The above shampoo conditions hair excellently in terms of combability, softness and elasticity and additionally gives fine hair excellent long lasting volume.

**Example 12**

| | % by weight |
|---|---|
| Sodium lauryl ether sulphate | 3.0 |
| Sodium lauryl ether carboxylate | 6.0 |
| Cocoyl polyglucoside | 3.0 |
| Cocoamphoacetate | 4.0 |
| Sodium cocyl glutamate | 2.0 |
| Cocoyl betaine | 1.0 |
| Polyquaternium-7 | 0.8 |
| Dimethicone | 0.5 |
| PEG-90 glyceryl isostearate | 3.0 |
| PPG- 12 | 0.6 |
| PPG-7 | 0.9 |
| Basic red 51 | 0.1 |
| Citric acid/sodium hydroxide | q.s. to pH 5.5 |
| Preservative, fragrance | q.s |
| Water | to 100 |

Above composition gives hair a red shine, in addition to the excellent conditioning effect.

**Example 13**

| | % by weight |
|---|---|
| Sodium lauryl ether sulfate | 9.0 |
| Laureth - 16 | 3.0 |
| Cocoyl betaine | 2.0 |
| Sodium lauroyl glutamate | 2.0 |
| Guarhydroxypropyltrimonium chloride | 1.0 |
| PEG-80 glyceryl cocoate | 4.0 |
| PPG-20 | 0.8 |
| Trimethyl pentaphenyl trisiloxane | 0.2 |
| Basic yellow 87 | 0.10 |
| Basic red 76 | 0.01 |
| Citric acid/sodium hydroxide | q.s. to pH 6.0 |
| Preservative, fragrance | q.s |

(continued)

| | % by weight |
|---|---|
| Water | to 100 |

Increase of volume and an excellent golden blonde shine was observed on light blond hair. Conditioning effect in terms of manageability and soft feeling upon touching is excellent.

**Example 14**

| | % by weight |
|---|---|
| Sodium lauryl ether sulfate | 3.0 |
| Sodium lauryl ether carboxylate | 7.0 |
| Laureth - 16 | 3.0 |
| Cocoyl betaine | 2.0 |
| Sodium lauroyl glutamate | 2.0 |
| Guarhydroxypropyltrimonium chloride | 1.0 |
| PEG-120 glyceryl stearate | 1.8 |
| PPG-7 | 1.8 |
| Dimethicone | 1.0 |
| Basic red 51 | 0.1 |
| Basic orange 31 | 0.05 |
| Citric acid/sodium hydroxide | q.s. to pH 5.7 |
| Preservative, fragrance | q.s |
| Water | to 100 |

An excellent red shine were observed on medium blond hair.

**Example 15**

| | % by weight |
|---|---|
| Sodium lauryl ether sulphate | 10.0 |
| Laureth - 16 | 3.0 |
| Cocoyl betaine | 2.0 |
| Sodium lauroyl glutamate | 2.0 |
| Polyquaternium-6 | 0.5 |
| Polysilicone-15 | 0.35 |
| Dimethicone | 0.5 |
| Ubiquinone | 0.1 |
| PEG-60 glyceryl isostearate | 4.0 |
| PPG-9 | 2.0 |
| Citric acid/sodium hydroxide | q.s. to pH 4.8 |
| Preservative, fragrance | q.s |
| Water | to 100 |

The above shampoo conditions hair excellently in terms of combability, softness, shine and elasticity and additionally gives fine hair excellent long lasting volume.

**Example 16**

| | % by weight |
|---|---|
| Sodium lauryl ether sulphate | 6.0 |
| Sodium lauryl ether carboxylate | 4.0 |
| Cocoyl glucoside | 3.0 |
| Cocamido propylbetaine | 2.0 |
| Sodium lauroyl glutamate | 2.0 |
| Polyquaternium-6 | 0.5 |
| Benzophenone-4 | 0.5 |
| Dimethicone | 0.5 |
| Ubiquinone | 0.1 |
| PEG-60 glyceryl isostearate | 4.0 |
| PPG-9 | 2.0 |
| Citric acid/sodium hydroxide | q.s. to pH 5.5 |
| Preservative, fragrance | q.s |
| Water | to 100 |

The above shampoo conditions hair excellently in terms of combability, shine, softness and elasticity and additionally gives fine hair excellent long lasting volume.

**Example 17**

| | % by weight |
|---|---|
| Sodium lauryl ether sulphate | 15.0 |
| Decyl glucoside | 3.0 |
| Cocamidopropyl betaine | 2.0 |
| Sodium cocyl glutamate | 2.0 |
| Polyquaternium-6 | 0.5 |
| Ethylhexyl methoxy cinnamate | 0.3 |
| Dimethicone | 0.5 |
| Ubiquinone | 0.1 |
| PEG-60 glyceryl isostearate | 4.0 |
| PPG-9 | 2.0 |
| Citric acid/sodium hydroxide | q.s. to pH 5.2 |
| Preservative, fragrance | q.s |
| Water | to 100 |

**Example 18**

| | % by weight |
|---|---|
| Sodium lauryl ether sulphate | 10.0 |
| Cocyl glucoside | 5.0 |
| Lauryl betaine | 4.0 |
| Sodium lauroyl glutamate | 2.0 |
| Polyquaternium-10 | 0.5 |
| Benzophenone-3 | 0.4 |

(continued)

| | % by weight |
|---|---|
| Dimethicone | 0.5 |
| PEG-60 glyceryl isostearate | 4.0 |
| PPG-9 | 2.0 |
| Citric acid/sodium hydroxide | q.s. to pH 5.4 |
| Preservative, fragrance | q.s |
| Water | to 100 |

**Claims**

1. Use of at least one ethoxylated monoglyceride of the following formula

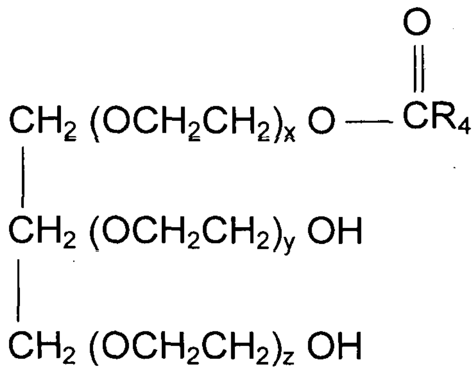

wherein $R_4$ is a saturated or unsaturated and branched or straight alkyl chain with a chain length of 7 to 21 C atoms and x+y+z has a value of 50 to 200, at a concentration of at least 1 % by weight, in a cleansing composition based on at least one sulphate type of anionic surfactant at a concentration of 2 to 25% by weight, and further comprising at least one amino acid surfactant of the following structure

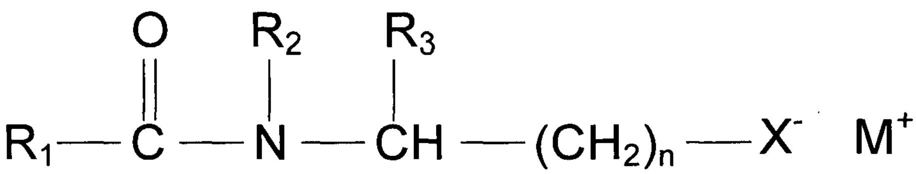

wherein $R_1$ is a saturated or unsaturated, straight or branched alkyl chain with 7 to 17 C atoms, $R_2$ is H or a methyl, $R_3$ is H, COO- $M^+$ or $CH_2COO^- M^+$, n is 0 to 2, X is $COO^-$ or $SO_3^-$ and $M^+$ is independent from each other H, sodium or potassium, at a concentration of 0.1 to 15%, by weight, all values calculated to total composition, as a shear thickening agent.

2. The use according to claim 1 **characterised in that** the cleansing composition comprises additionally at least one non-ionic surfactant.

3. The use according to claim 2 **characterised in that** the composition comprises at least one non-ionic surfactant according to the general formula

$$R_6\text{-}O\text{-}(R_4O)_n\text{-}Z_x,$$

wherein $R_6$ is an alkyl group with 8 to 18 carbon atoms, $R_4$ is an ethylene or propylene group, Z is a saccharide group with 5 to 6 carbon atoms, n is a number from 0 to 10 and x is a number between 1 and 5.

**4.** The use according to any of the preceding claims **characterised in that** the composition comprises additionally at least one amphoteric surfactant.

**5.** The use according to claim 4 **characterised in that** at least one amphoteric surfactant is selected from betaines, amidoalkyl betaines and sulfobetaines, and their mixtures.

**6.** The use according to any of the proceedings claims **characterised in that** at least one amino acid surfactant is selected from

i- taurate surfactants according to the general formula

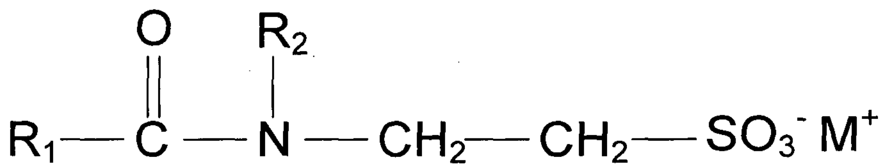

wherein $R_1$ is preferably a saturated or unsaturated, straight or branched alkyl chain with 7 to 17 C atoms, and more preferably 9 to 13 C atoms, $R_2$ is H or methyl, and M is H, sodium or potassium,
ii- glutamate surfactants are according to the general formula

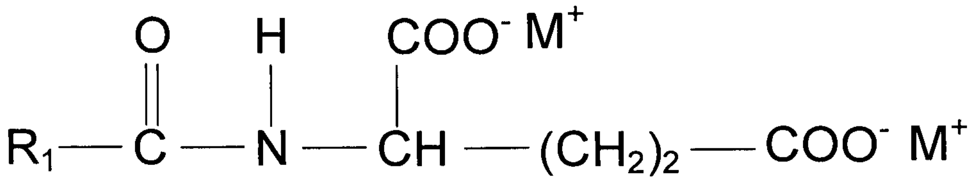

wherein $R_1$ is preferably a saturated or unsaturated, straight or branched alkyl chain with 7 to 17 C atoms, and more preferably 9 to 13 C atoms, and M is independent from each other H, sodium or potassium,
iii- alanine or alaninate surfactants according to the general formula

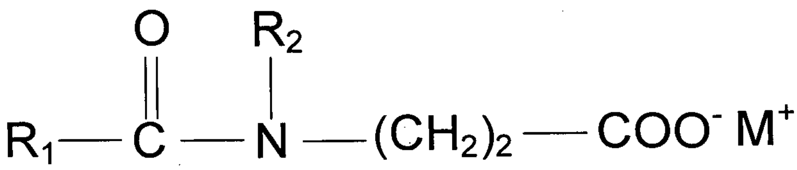

wherein $R_1$ is preferably a saturated or unsaturated, straight or branched alkyl chain with 7 to 17 C atoms, and more preferably 9 to 13 C atoms, $R_2$ is H or methyl and M is H, sodium or potassium,
iv- glycine surfactants according to the general formula

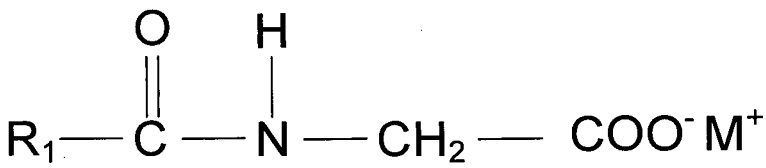

wherein $R_1$ is preferably a saturated or unsaturated, straight or branched alkyl chain with 7 to 17 C atoms, and more preferably 9 to 13 C atoms, and M is H, sodium or potassium,
v- sarcosinate surfactants according to the general formula

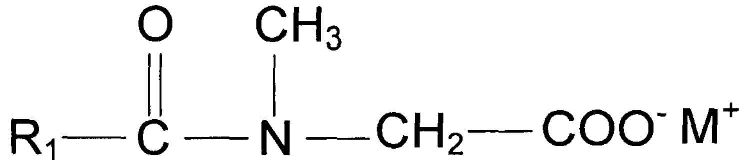

wherein $R_1$ is preferably a saturated or unsaturated, straight or branched alkyl chain with 7 to 17 C atoms, and more preferably 9 to 13 C atoms and M is H, sodium or potassium, and

vi- aspartate surfactants according to the general formula

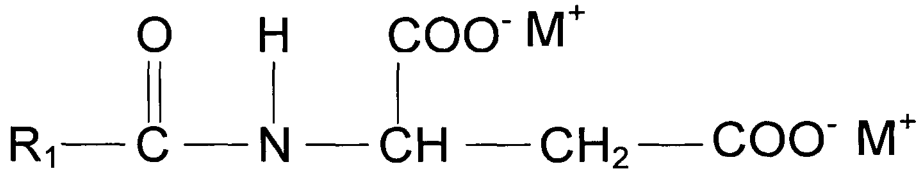

wherein $R_1$ is preferably a saturated or unsaturated, straight or branched alkyl chain with 7 to 17 C atoms, and more preferably 9 to 13 C atoms and M is independent from each other H, sodium or potassium.

**7.** The use according to claim 6 **characterised in that** at least one amino acid surfactant is selected from potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, sodium methyl stearoyl taurate, dipotassium capryloyl glutamate, dipotassium undecylenoyl glutamate, disodium capryloyl glutamate, disodium cocoyl glutamate, disodium lauroyl glutamate, disodium stearoyl glutamate, disodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, sodium undecylenoyl glutamate, cocoyl methyl ß-alanine, lauroyl ß-alanine, lauroyl methyl ß-alanine, myristoyl β-alanine, potassium lauroyl methyl β-alanine, sodium cocoyl alaninate, sodium cocoyl methyl β-alanine and sodium myristoyl methyl ß-alanine palmitoyl glycine, sodium lauroyl glycine, sodium cocoyl glycine, sodium myristoyl glycine, potassium lauroyl glycine, potassium cocoyl glycine, potassium lauroyl sarcosinate, potassium cocoyl sarcosinate, sodium cocoyl sarcosinate, sodium lauroyl sarcosinate, sodium myristoyl sarcosinate, and sodium palmitoyl sarcosinate and mixtures thereof. Preferred are potassium lauroyl sarcosinate, potassium cocoyl sarcosinate, sodium cocoyl sarcosinate, sodium lauroyl sarcosinate, sodium lauroyl aspartate, sodium myristoyl aspartate, sodium cocoyl aspartate, sodium caproyl aspartate, disodium lauroyl aspartate, disodium myristoyl aspartate, disodium cocoyl aspartate, disodium caproyl aspartate, potassium lauroyl aspartate, potassium myristoyl aspartate, potassium cocoyl aspartate, potassium caproyl aspartate, dipotassium lauroyl aspartate, dipotassium myristoyl aspartate, dipotassium cocoyl aspartate, and dipotassium caproyl aspartate and mixtures thereof.

**8.** The use according to any of the preceding claims **characterised in that** at least one ethoxylated monoglyceride is selected from PEG-78 glyceryl cocoate, PEG-80 glyceryl cocoate, PEG-50 glyceryl isostearate, PEG-60 glyceryl isostearate, PEG-90 glyceryl isostearate, PEG-60 glyceryl stearate, PEG-120 glyceryl stearate, PEG-200 glyceryl stearate, PEG-80 glyceryl tallowate, PEG-82 glyceryl tallowate, PEG-130 glyceryl tallowate and PEG-200 glyceryl tallowate, and mixtures thereof.

**9.** The use according to any of the preceding claims **characterised in that** the composition comprises at least one (poly)propylene glycol according to the following formula

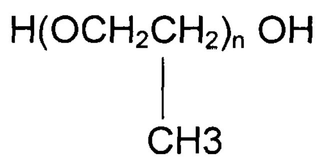

wherein n has a value between 1 and 70.

10. The use according to any of the preceding claims **characterised in that** the composition comprises at least one conditioning agent.

11. The use according to claim 10 **characterised in that** the composition comprises at least one cationic polymer as conditioning agent.

12. The use according to claims 10 and 11 **characterised in that** the composition comprises oily substances as conditioning agent selected from silicone oils, either volatile or non-volatile, natural and synthetic oils.

13. The use according to any of the preceding claims **characterised in that** the composition comprises at least one UV filter.

14. The use according to any of the preceding claims **characterised in** the composition comprises at least one direct dye.

**Patentansprüche**

1. Verwendung mindestens eines ethoxylierten Monoglycerids der folgenden Formel

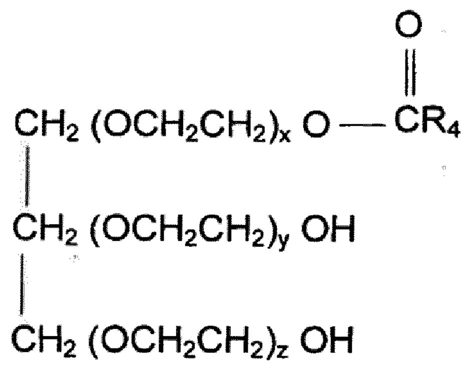

wobei $R_4$ eine gesättigte oder ungesättigte und verzweigte oder geradkettige Alkylkette mit einer Kettenlänge von 7 bis 21 C-Atomen ist und x + y + z einen Wert von 50 bis 200 aufweist, in einer Konzentration von mindestens 1 Gewichts-% in einer Reinigungszusammensetzung, die auf mindestens einem anionischen Tensid des Sulfattyps in einer Konzentration von 2 Gewichts-% bis 25 Gewichts-% basiert und ferner mindestens ein Aminosäuretensid der folgenden Struktur

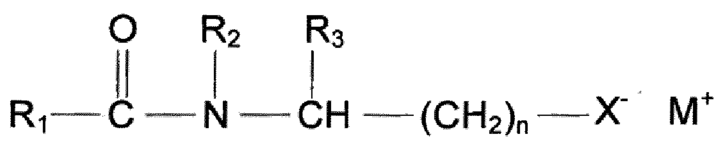

wobei $R_1$ für eine gesättigte oder ungesättigte, geradkettige oder verzweigte Alkylkette mit 7 bis 17 C-Atomen steht, $R_2$ für H oder eine Methylgruppe steht, $R_3$ für H, COO- $M^+$ oder $CH_2COO^-$ $M^+$ steht, n 0 bis 2 ist, X für COO-oder $SO_3^-$ steht und M unabhängig für H, Natrium oder Kalium steht, in einer Konzentration von 0,1 Gewichts-% bis 15 Gewichts-%, wobei alle Werte auf die Gesamtzusammensetzung bezogen sind, als ein Scherverfestigungsmittel aufweist.

2. Verwendung nach Anspruch 1, **dadurch gekennzeichnet, dass** die Reinigungszusammensetzung zusätzlich mindestens ein nichtionisches Tensid aufweist.

3. Verwendung nach Anspruch 2, **dadurch gekennzeichnet, dass** die Zusammensetzung mindestens ein nichtionisches Tensid der allgemeinen Formel

$R_6$-O-$(R_4O)_n$-$Z_x$,

aufweist, wobei $R_6$ für eine Alkylgruppe mit 8 bis 18 Kohlenstoffatomen steht, $R_4$ für eine Ethylen- oder Propylengruppe steht, Z für eine Saccharidgruppe mit 5 bis 6 Kohlenstoffatomen steht, n eine Zahl von 0 bis 10 ist und x eine Zahl von 1 bis 5 ist.

4. Verwendung nach einem der vorhergehenden Ansprüche, **dadurch gekennzeichnet, dass** die Zusammensetzung zusätzlich mindestens ein amphoteres Tensid aufweist.

5. Verwendung nach Anspruch 4, **dadurch gekennzeichnet, dass** mindestens ein amphoteres Tensid aus Betainen, Amidoalkylbetainen und Sulfobetainen und deren Gemischen ausgewählt ist.

6. Verwendung nach einem der vorhergehenden Ansprüche, **dadurch gekennzeichnet, dass** mindestens ein Aminosäuretensid ausgewählt ist aus

i- Taurattensiden der allgemeinen Formel

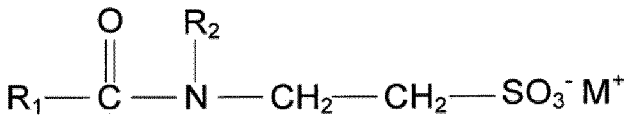

wobei $R_1$ vorzugsweise für eine gesättigte oder ungesättigte, geradkettige oder verzweigte Alkylkette mit 7 bis 17 C-Atomen, insbesondere 9 bis 13 C-Atomen steht, $R_2$ für H oder Methyl steht und M für H, Natrium oder Kalium steht,
ii- Glutamattensiden der allgemeinen Formel

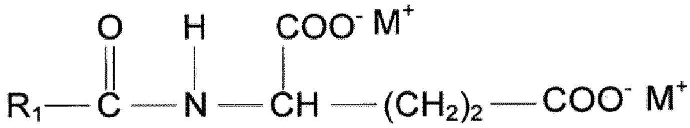

wobei $R_1$ vorzugsweise für eine gesättigte oder ungesättigte, geradkettige oder verzweigte Alkylkette mit 7 bis 17 C-Atomen, insbesondere 9 bis 13 C-Atomen steht und M unabhängig für H, Natrium oder Kalium steht,
iii- Alanin- oder Alaninattensiden der allgemeinen Formel

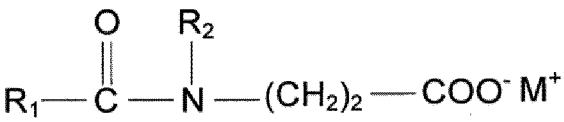

wobei $R_1$ vorzugsweise für eine gesättigte oder ungesättigte, geradkettige oder verzweigte Alkylkette mit 7 bis 17 C-Atomen, insbesondere 9 bis 13 C-Atomen steht, $R_2$ für H oder Methyl steht und M für H, Natrium oder Kalium steht,
iv- Glycintensiden der allgemeinen Formel

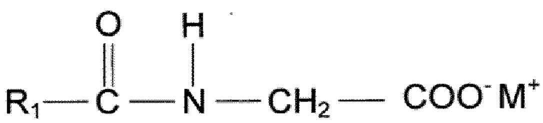

wobei $R_1$ vorzugsweise für eine gesättigte oder ungesättigte, geradkettige oder verzweigte Alkylkette mit 7 bis 17 C-Atomen, insbesondere 9 bis 13 C-Atomen steht und M für H, Natrium oder Kalium steht,
v- Sarcosinattensiden der allgemeinen Formel

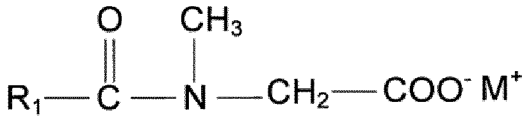

wobei $R_1$ vorzugsweise für eine gesättigte oder ungesättigte, geradkettige oder verzweigte Alkylkette mit 7 bis 17 C-Atomen, insbesondere 9 bis 13 C-Atomen steht und M für H, Natrium oder Kalium steht, und
vi- Aspartattensiden der allgemeinen Formel

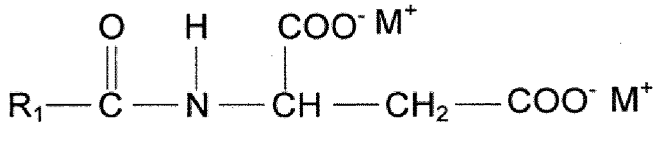

wobei $R_1$ vorzugsweise für eine gesättigte oder ungesättigte, geradkettige oder verzweigte Alkylkette mit 7 bis 17 C-Atomen, insbesondere 9 bis 13 C-Atomen steht und M unabhängig für H, Natrium oder Kalium steht.

**7.** Verwendung nach Anspruch 6, **dadurch gekennzeichnet, dass** mindestens ein Aminosäuretensid aus Kaliumcocoyltaurat, Kaliummethylcocoyltaurat, Natriumcaproylmethyltaurat, Natriumcocoyltaurat, Natriumlauroyltaurat, Natriummethylcocoyltaurat, Natriummethyllauroyltaurat, Natriummethylmyristoyltaurat, Natriummethyloleoyltaurat, Natriummethylpalmitoyltaurat, Natriummethylstearoyltaurat, Dikaliumcapryloylglutamat, Dikaliumundecylenoylglutamat, Dinatriumcapryloylglutamat, Dinatriumcocoylglutamat, Dinatriumlauroylglutamat, Dinatriumstearoylglutamat, Dinatriumundecylenoylglutamat, Kaliumcapryloylglutamat, Kaliumcocoylglutamat, Kaliumlauroylglutamat, Kaliummyristoylglutamat, Kaliumstearoylglutamat, Kaliumundecylenoylglutamat, Natriumcapryloylglutamat, Natriumcocoylglutamat, Natriumlauroylglutamat, Natriummyristoylglutamat, Natriumolivoylglutamat, Natriumpalmitoylglutamat, Natriumstearoylglutamat, Natriumundecylenoylglutamat, Cocoylmethyl-β-alanin, Lauroyl-β-alanin, Lauroylmethyl-β-alanin, Myristoyl-β-alanin, Kaliumlauroylmethyl-β-alanin, Natriumcocoylalaninat, Natriumcocoylmethyl-β-alanin und Natriummyristoylmethyl-β-alaninpalmitoylglycin, Natriumlauroylglycin, Natriumcocoylglycin, Natriummyristoylglycin, Kaliumlauroylglycin, Kaliumcocoylglycin, Kaliumlauroylsarcosinat, Kaliumcocoylsarcosinat, Natriumcocoylsarcosinat, Natriumlauroylsarcosinat, Natriummyristoylsarcosinat und Natriumpalmitoylsarcosinat und Gemischen dieser ausgewählt ist, vorzugsweise aus Kaliumlauroylsarcosinat, Kaliumcocoylsarcosinat, Natriumcocoylsarcosinat, Natriumlauroylsarcosinat, Natriumlauroylaspartat, Natriummyristoylaspartat, Natriumcocoylaspartat, Natriumcaproylaspartat, Dinatriumlauroylaspartat, Dinatriummyristoylaspartat, Dinatriumcocoylaspartat, Dinatriumcaproylaspartat, Kaliumlauroylaspartat, Kaliummyristoylaspartat, Kaliumcocoylaspartat, Kaliumcaproylaspartat, Dikaliumlauroylaspartat, Dikaliummyristoylaspartat, Dikaliumcocoylaspartat und Dikaliumcaproylaspartat und Gemischen dieser.

**8.** Verwendung nach einem der vorhergehenden Ansprüche, **dadurch gekennzeichnet, dass** mindestens ein ethoxyliertes Monoglycerid aus PEG-78-Glycerylcocoat, PEG-80-Glycerylcocoat, PEG-50-Glycerylisostearat, PEG-60-Glycerylisostearat, PEG-90-Glycerylisostearat, PEG-60-Glycerylstearat, PEG-120-Glycerylstearat, PEG-200-Glycerylstearat, PEG-80-Glyceryltalgat, PEG-82-Glyceryltalgat, PEG-130-Glyceryltalgat und PEG-200-Glyceryltalgat und Gemischen dieser ausgewählt ist.

**9.** Verwendung nach einem der vorhergehenden Ansprüche, **dadurch gekennzeichnet, dass** die Zusammensetzung mindestens ein (Poly)propylenglykol der folgenden Formel

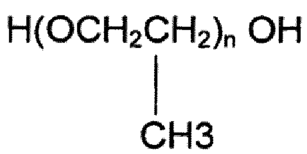

aufweist, wobei n einen Wert von 1 bis 70 aufweist.

**10.** Verwendung nach einem der vorhergehenden Ansprüche, **dadurch gekennzeichnet, dass** die Zusammensetzung mindestens einen Konditionierer aufweist.

11. Verwendung nach Anspruch 10, **dadurch gekennzeichnet, dass** die Zusammensetzung mindestens ein kationisches Polymer als Konditionierer aufweist.

12. Verwendung nach Anspruch 10 und 11, **dadurch gekennzeichnet, dass** die Zusammensetzung ölige Substanzen als Konditionierer aufweist, ausgewählt aus Silikonölen, entweder flüchtig oder nichtflüchtig, natürlichen und synthetischen Ölen.

13. Verwendung nach einem der vorhergehenden Ansprüche, **dadurch gekennzeichnet, dass** die Zusammensetzung mindestens einen UV-Filter aufweist.

14. Verwendung nach einem der vorhergehenden Ansprüche, **dadurch gekennzeichnet, dass** die Zusammensetzung mindestens einen Direktfarbstoff aufweist.

**Revendications**

1. Utilisation d'au moins un monoglycéride éthoxylé selon la formule suivante

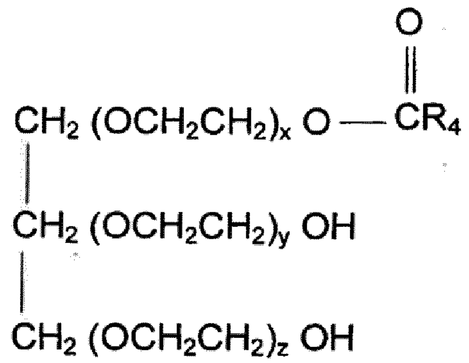

dans laquelle $R_4$ est une chaîne alkyle saturée ou non saturée, et ramifiée ou linéaire, avec une longueur de chaîne de 7 à 21 atomes de C, et la somme x + y + z a une valeur entre 50 et 200, à une concentration d'au moins 1 % en poids, dans une composition nettoyante basée sur au moins un tensioactif de type sulfate à une concentration entre 2 et 25 % en poids, et qui comprend en outre au moins un tensioactif de type acide aminé selon la structure suivante

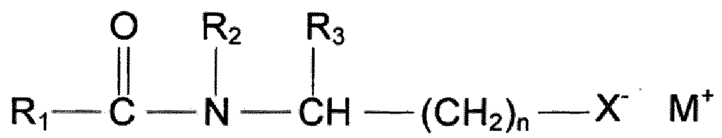

dans laquelle $R_1$ est une chaîne alkyle saturée ou non saturée, ramifiée ou linéaire, avec une longueur de la chaîne de 7 à 17 atomes de C, $R_2$ représente un atome H ou un groupe méthyle, $R_3$ représente un atome H, un groupe $COO^- M^+$ ou $CH_2COO^- M^+$, n est un nombre entre 0 et 2, X représente une fonction $COO^-$ ou $SO_3^-$ et les M sont indépendamment les uns des autres un atome H, de sodium ou de potassium, à une concentration entre 0,1 et 15 % en poids, toutes les valeurs étant calculées par rapport à la composition totale, en tant qu'agent de rhéopexie.

2. Utilisation selon la revendication 1, **caractérisée en ce que** la composition nettoyante comprend à titre complémentaire au moins un tensioactif non ionique.

3. Utilisation selon la revendication 2, **caractérisée en ce que** la composition comprend au moins un tensioactif non ionique selon la formule générale

$$R_6\text{-O-}(R_4O)_n\text{-}Z_x,$$

dans laquelle $R_6$ est un groupe alkyle avec entre 8 et 18 atomes de carbone, $R_4$ est un groupement éthylène ou propylène, Z est un groupement saccharide avec 5 à 6 atomes de carbone, n est un nombre entre 0 et 10 et x est

un nombre entre 1 et 5.

**4.** Utilisation selon l'une quelconque des revendications précédentes, **caractérisée en ce que** la composition comprend à titre complémentaire au moins un tensioactif amphotère.

**5.** Utilisation selon la revendication 4, **caractérisée en ce qu'**au moins un tensioactif amphotère est choisi parmi les bétaïnes, les amidoalkyl bétaïnes et les sulfobétaïnes, et les mélanges de celles-ci.

**6.** Utilisation selon l'une quelconque des revendications précédentes, **caractérisée en ce qu'**au moins un tensioactif de type acide aminé est choisi parmi

i- les tensioactifs de type taurate selon la formule générale

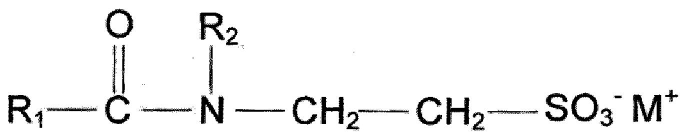

dans laquelle $R_1$ est de préférence une chaine alkyle saturée ou non saturée, linéaire ou ramifiée, de 7 à 17 atomes de C, et de manière plus préférable de 9 à 13 atomes de C, $R_2$ représente un atome H ou un groupe méthyle, et M est un atome H, de sodium ou de potassium,
ii- les tensioactifs de type glutamate selon la formule générale

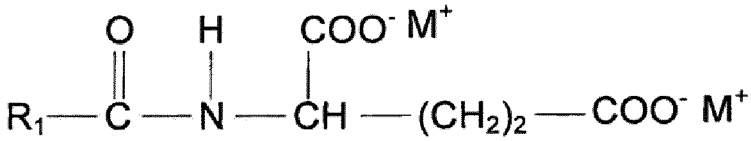

dans laquelle $R_1$ est de préférence une chaine alkyle saturée ou non saturée, linéaire ou ramifiée, de 7 à 17 atomes de C, et de manière plus préférable de 9 à 13 atomes de C, et les M sont indépendamment les uns des autres un atome H, de sodium ou de potassium,
iii- les tensioactifs de type alanine ou alaninate selon la formule générale

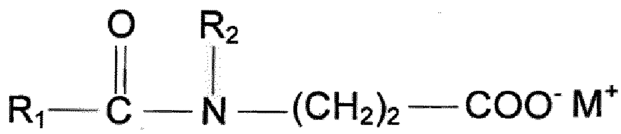

dans laquelle $R_1$ est de préférence une chaine alkyle saturée ou non saturée, linéaire ou ramifiée, de 7 à 17 atomes de C, et de manière plus préférable de 9 à 13 atomes de C, $R_2$ représente un atome H ou un groupe méthyle, et M est un atome H, de sodium ou de potassium,
iv- les tensioactifs de type glycine selon la formule générale

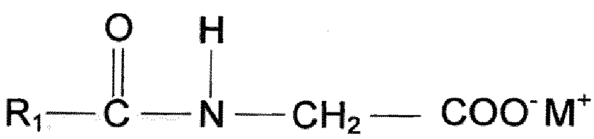

dans laquelle $R_1$ est de préférence une chaine alkyle saturée ou non saturée, linéaire ou ramifiée, de 7 à 17 atomes de C, et de manière plus préférable de 9 à 13 atomes de C, et M est un atome H, de sodium ou de potassium,
v- les tensioactifs de type sarcosinate selon la formule générale

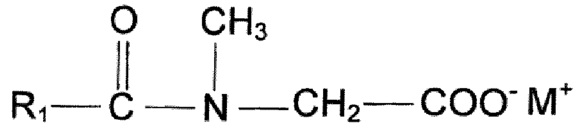

dans laquelle $R_1$ est de préférence une chaine alkyle saturée ou non saturée, linéaire ou ramifiée, de 7 à 17 atomes de C, et de manière plus préférable de 9 à 13 atomes de C, et M est un atome H, de sodium ou de potassium, et

vi- les tensioactifs de type aspartate selon la formule générale

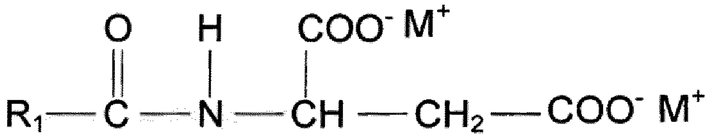

dans laquelle $R_1$ est de préférence une chaine alkyle saturée ou non saturée, linéaire ou ramifiée, de 7 à 17 atomes de C, et de manière plus préférable de 9 à 13 atomes de C, et les M sont indépendamment les uns des autres un atome H, de sodium ou de potassium.

**7.** Utilisation selon la revendication 6 **caractérisée en ce qu'**au moins un tensioactif de type acide aminé est choisi à partir du cocoyl taurate de potassium, du méthyl cocoyl taurate de potassium, du caproyl méthyl taurate de sodium, du cocoyl taurate de sodium, du lauroyl taurate de sodium, du méthyl cocoyl taurate de sodium, du méthyl lauroyl taurate de sodium, du méthyl myristoyl taurate de sodium, du méthyl oléoyl taurate de sodium, du méthyl palmitoyl taurate de sodium, du méthyl stéaroyl taurate de sodium, du capryloyl glutamate dipotassique, de l'undécylénoyl glutamate dipotassique, du capryloyl glutamate disodique, du cocoyl glutamate disodique, du lauroyl glutamate disodique, du stéaroyl glutamate disodique, de l'undécylénoyl glutamate disodique, du capryloyl glutamate de potassium, du cocoyl glutamate de potassium, du lauroyl glutamate de potassium, du myristoyl glutamate de potassium, du stéaroyl glutamate de potassium, de l'undécylénoyl glutamate de potassium, du capryloyl glutamate de sodium, du cocoyl glutamate de sodium, du lauroyl glutamate de sodium, du myristoyl glutamate de sodium, du olivoyl glutamate de sodium, du palmitoyl glutamate de sodium, du stéaroyl glutamate de sodium, de l'undécylénoyl glutamate de sodium, de la cocoyl méthyl β alanine, de la lauroyl β alanine, de la lauroyl méthyl β alanine, de la myristoyl β alanine, de la lauroyl méthyl β alanine de potassium, du cocoyl alaninate de sodium, de la cocoyl méthyl β alanine de sodium et de la myristoyl méthyl β alanine palmitoyl glycine de sodium, de la lauroyl glycine de sodium, de la cocoyl glycine de sodium, de la myristoyl glycine de sodium, de la lauroyl glycine de potassium, de la cocoyl glycine de potassium, du lauroyl sarcosinate de potassium, du cocoyl sarcosinate de potassium, du cocoyl sarcosinate de sodium, du lauroyl sarcosinate de sodium, du myristoyl sarcosinate de sodium, et du palmitoyl sarcosinate de sodium et les mélanges de ceux-ci. De manière préférable, il s'agit du lauroyl sarcosinate de potassium, du cocoyl sarcosinate de potassium, du cocoyl sarcosinate de sodium, du lauroyl sarcosinate de sodium, du lauroyl aspartate de sodium, du myristoyl aspartate de sodium, du cocoyl aspartate de sodium, du caproyl aspartate de sodium, du lauroyl aspartate disodique, du myristoyl aspartate disodique, du cocoyl aspartate disodique, du caproyl aspartate disodique, du lauroyl aspartate de potassium, du myristoyl aspartate de potassium, du cocoyl aspartate de potassium, du caproyl aspartate de potassium, du lauroyl aspartate dipotassique, du myristoyl aspartate dipotassique, du cocoyl aspartate dipotassique, et du caproyl aspartate dipotassique et les mélanges de ceux-ci.

**8.** Utilisation selon l'une quelconque des revendications précédentes, **caractérisée en ce qu'**au moins un monoglycéride éthoxylé est choisi parmi le glycéryl cocoate de PEG-78, le glycéryl cocoate de PEG-80, le glycéryl isostéarate de PEG-50, le glycéryl isostéarate de PEG-60, le glycéryl isostéarate de PEG-90, le glycéryl stéarate de PEG-60, le glycéryl stéarate de PEG-120, le glycéryl stéarate de PEG-200, le glycéryl tallowate de PEG-80, le glycéryl tallowate de PEG-82, le glycéryl tallowate de PEG-130 et le glycéryl tallowate de PEG-200, et les mélanges de ceux-ci.

**9.** Utilisation selon l'une quelconque des revendications précédentes, **caractérisée en ce que** la composition comprend au moins un (poly)propylène glycol selon la formule suivante

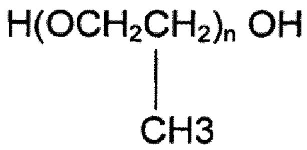

dans laquelle n a une valeur entre 1 et 70.

10. Utilisation selon l'une quelconque des revendications précédentes, **caractérisée en ce que** la composition comprend au moins un agent de conditionnement.

11. Utilisation selon la revendication 10, **caractérisée en ce que** la composition comprend au moins un polymère cationique en tant qu'agent de conditionnement.

12. Utilisation selon les revendications 10 et 11, **caractérisée en ce que** la composition comprend des substances huileuses choisies parmi des huiles silicones, soit volatiles soit non volatiles, des huiles naturelles et synthétiques en tant qu'agent de conditionnement.

13. Utilisation selon l'une quelconque des revendications précédentes, **caractérisée en ce que** la composition comprend au moins un filtre UV.

14. Utilisation selon l'une quelconque des revendications précédentes, **caractérisée en ce que** la composition comprend au moins un colorant direct.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03013459 A2
- WO 9740125 A1
- WO 03063818 A1
- WO 2004024110 A1
- WO 03013467 A1
- EP 70074 A
- EP 358216 A
- DE 2521960
- DE 2811010
- DE 3044738
- DE 3217059
- EP 337354 A
- EP 524612 A
- EP 640643 A
- US 3927199 A
- WO 2005065632 A1


### Non-patent literature cited in the description


- Das Rheologie Handbuch. Vincentz Network GmbH, 2006, 246-250